# EUROPEAN PATENT APPLICATION

(11) **EP 2 589 384 A1**
(43) Date of publication of application: **08.05.2013**
(21) Application number: 13151040.6
(22) Date of filing: 29.08.2005
(51) Int. Cl.: A61K 31/44, A61K 9/16, A61P 35/00

(54) **New Pharmaceutical Compositions for the Treatment of Hyper-Proliferative Disorders**

(30) Priority: 27.08.2004 US 604752 P
(62) Divisional of application: 05792486.2
(71) Applicant: Bayer Healthcare LLC, Tarrytown, NY 10591-5097 (US)
(72) Inventor: Skrabs, Susanne, 13189 Berlin (DE); Dumas, Jacques, Bethany CT 06524-0000, CT Connecticut (US); Ehrlich, Paul, West Haven CT 06516-4175, CT Connecticut (US)
(74) Representative: BIP Patents

(57) **Abstract**

This invention relates to novel pharmaceutical compositions comprising a solid dispersion of the compound of Formula I below, to processes for preparing these novel pharmaceutical compositions and to their use for treating hyper-proliferative disorders, such as cancer, either as a sole agent or in combination with other therapies.
Formula I is as follows:

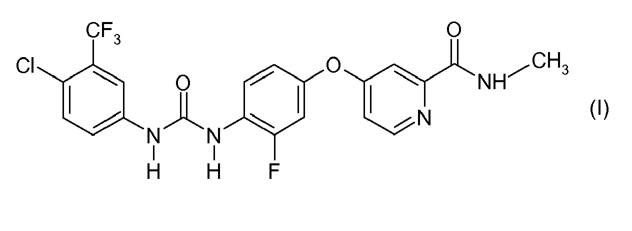

## Description

### Field of the Invention

This application claims the benefit of the filing date of U.S. Provisional Application Serial No. 60/604,752 filed August 27, 2004, which is incorporated by reference herein.

This invention relates to novel pharmaceutical compositions, to processes for preparing these novel pharmaceutical compositions and to their use for treating hyper-proliferative disorders, such as cancer, either as a sole agent or in combination with other therapies.

### Background of the Invention

Diarylureas are a class of serine-threonine kinase inhibitors as well as tyrosine kinase inhibitors known in the art. The following publications illustrate their utility as active ingredient in pharmaceutical compositions for the treatment of hyper-proliferative diseases, such as cancer:
Smith et al., Bioorg. Med. Chem. Lett. 2001, 11, 2775-2778.
Lowinger et al., Clin. Cancer Res. 2000, 6(suppl.), 335.
Lyons et al., Endocr.-Relat. Cancer 2001, 8, 219-225.
Riedl et al., Book of Abstracts, 92nd AACR Meeting, New Orleans, LA, USA, abstract 4956*.*
Khire et al., Book of Abstracts, 93rd AACR Meeting, San Francisco, CA, USA, abstract 4211*.*
Lowinger et al., Curr. Pharm. Design 2002, 8, 99-110.
Carter et al., Book of Abstracts, 92nd AACR Meeting, New Orleans, LA, USA, abstract 4954*.*
Vincent et al., Book of Abstracts, 38th ASCO Meeting, Orlando, FL, USA, abstract 1900*.*
Hilger et al., Book of Abstracts, 38th ASCO Meeting, Orlando, FL, USA, abstract 1916*.*
Moore et al., Book of Abstracts, 38th ASCO Meeting, Orlando, FL, USA, abstract 1816*.*
Strumberg et al., Book of Abstracts, 38th ASCO Meeting, Orlando, FL, USA, abstract 121*.*
Omega-Carboxyaryl diphenyl ureas are disclosed in WO00/42012 (published July 20, 2000), WO00/41698 (published July 20, 2000), and in the following published U.S. applications:
   US2002-0165394-A1, published November 7, 2002,
   US2001-003447-A1, published October 25, 2001,
   US2001-0016659-A1, published August 23, 2001,
   US2002-013774-A1, published September 26, 2002,
and copending U.S. applications:
   09/758,547, filed January 12, 2001,
   09/889,227, filed July 12, 2001,
   09/993,647, filed November 27, 2001,
   10/042,203, filed January 11, 2002 and
   10/071,248, filed February 11, 2002

In particular, it has been discovered that the diphenyl urea of Formula I, also referred as 4{4-[3-(4-chloro-3-trifluoromethylphenyl)-ureido]-3-fluorophenoxy}-pyridine-2-carboxylic acid methyl amide is a potent inhibitor of raf, VEGFR-2, p38, and PDGFR kinases. These enzymes are all molecular targets of interest for the treatment of hyper-proliferative diseases, including cancer. Therefore, the compound of Formula I will be used as medicine for the treatment of the above mentioned diseases.

The preferred route of drug administration is through the oral cavity. This route provides the greatest comfort and convenience of dosing. The bioavailability achieved after oral administration is a measure for the potential usefulness of an oral dosage form of a drug. Bioavailability after oral application depends on several factors, such as solubility of the active in aqueous media, dose strength, dissolution of the dosage form, absorption throughout the gastrointestinal tract and first pass effect.

Therefore solid pharmaceutical compositions for oral application containing the compound of Formula I, which result in improved dissolution, absorption and exposure in mammals, improved inter-patient variability, and overall improved efficacy in the clinic are desired.

It has now been discovered that the target of improved dissolution, superior absorption and increased bioavailability and other needs, which will become apparent to one skilled in the art, are met by the present invention, which is described in detail below.

### Description of the Invention

The use of a novel pharmaceutical composition comprising a solid dispersion of the compound of Formula I will provide significant advantages in the clinic.

Formula I is as follows:

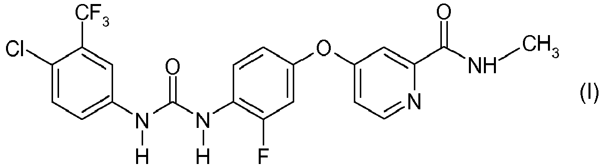

The term "the compound of Formula I", or "the compound of this invention" does not only refer to 4 {4-[3-(4-chloro-3-trifluoromethylphenyl)-ureido]-3-fluorophenoxy}-pyridine-2-carboxylic acid methyl amide as depicted in Formula I, but also refers to its solvates, hydrates, pharmaceutically acceptable salts, or a combination thereof.

The present invention pertains to
(i) novel pharmaceutical compositions containing the compound of Formula I in the form of a solid dispersion, which includes solid solutions, glass solutions, glass suspensions, amorphous precipitations in a crystalline carrier, eutectics or monotecics, compound or complex formation and combinations thereof.
(ii) processes for preparing these novel pharmaceutical compositions
(iii) the use of these compositions for the treatment of hyper-proliferative diseases, such as cancer, either as a sole agent, or in combination with other therapies.

In the following, the different types of solid dispersions (solid solutions, glass solutions, glass suspensions, amorphous precipitations in a crystalline carrier, eutectics or monotecics, compound or complex formation and combinations thereof) are collectively referred to as solid dispersion.

A pharmaceutical composition according to this invention comprises of a solid dispersion comprising at least the compound of Formula I and a pharmaceutically acceptable matrix.

The term "matrix" or "matrix agents" as used herein refers to both polymeric excipients, non-polymeric excipients and combinations thereof, capable of dissolving or dispersing the compound of formula I.

An aspect of the invention of particular interest is a pharmaceutical composition comprising a solid dispersion, wherein the matrix comprises a pharmaceutically acceptable polymer, such as, for example, polyvinylpyrrolidone, vinylpyrrolidone/ vinylacetate copolymer, crospovidone, polyalkylene glycol (e.g. polyethylene glycol), polyethylenoxide, poloxamer, hydroxyalkyl cellulose (e.g. hydroxypropyl cellulose), hydroxyalkyl methyl cellulose (e.g. hydroxypropyl methyl cellulose), carboxymethyl cellulose, sodium carboxymethyl cellulose, ethyl cellulose, cellulose succinates (e.g. cellulose acetate succinate and hydroxypropyl methyl cellulose acetate succinate), cellulose phthalates (e.g. cellulose acetate phthalate and hydroxypropyl methyl cellulose phthalate), polymethacrylates (e.g. Eudragit^{®} types), polyhydroxyalkylacrylates, polyhydroxyalkylmethacrylates, polyacrylates, polyvinyl alcohol, polyvinyl acetate, vinyl alcohol/vinyl acetate copolymer, xanthan gum, galactomannanes, carrageenan, chitosan, chitin, alginic acid and its salts, polylactides, dextrins, starch and starch derivatives, proteins and combinations thereof.

Another aspect of the invention is a pharmaceutical composition comprising a solid dispersion, wherein the matrix comprises a sugar and/or sugar alcohol and/or cyclodextrin, such as, for example sucrose, lactose, fructose, maltose, raffinose, sorbitol, lactitol, mannitol, maltitol, erythritol, threitol, adonitol, arabitol, xylitol, dulcitol, inositol, trehalose, isomalt, inulin, maltodextrin, β-cyclodextrin, hydroxypropyl-β-cyclodextrin, sulfobutyl ether cyclodextrin or combinations thereof.

Additional suitable excipients that are useful in the formation of the matrix of the solid dispersion include, but are not limited to alcohols, organic acids, organic bases, salts, amino acids, peptides, phospholipids, lipids (e.g. mono-, di- and triglycerides), fatty acids, fatty alcohols, waxes, fatty acid esters, polyoxyethylene sorbitan fatty acid esters, polyglycolized glycerides, sucrose esters, urea and combinations thereof.

The solid dispersion of the compound of Formula I in the matrix may contain certain additional pharmaceutical acceptable ingredients, such as carriers, surfactants, fillers, disintegrants, adsorbants, recrystallization inhibitors, plasticizers, fluxing agents, defoamers, antioxidants, detackifier, pH-modifiers, glidants and lubricants.

A carrier according to this invention is an excipient, which becomes loaded with a mixture, comprising of at least the matrix agent and the compound of this invention, during the manufacturing process of the solid dispersion, for example by hot melt extrusion, hot melt coating, prilling, congealing, solvent evaporation processes (e.g. layering, coating, granulation), and thus becomes an integral part of the solid dispersion.

In a preferred embodiment of this invention, the matrix comprises a water soluble polymer.

In another preferred embodiment at least one from the group of polyvinylpyrrolidone, copovidone, hydroxypropyl cellulose, hydroxypropyl methyl cellulose, polyethylene glycol and polyethylene oxide is used as matrix agent in the solid dispersion.

In another preferred embodiment polyvinylpyrrolidone is used as matrix agent.

An embodiment of particular interest comprises the compound of Formula I (calculated as solvent-free base) and polyvinylpyrrolidone in a weight ratio of 1:0.5 to 1:20.

Another preferred embodiment comprises hydroxypropyl cellulose as matrix agent.

An embodiment of particular interest comprises the compound of Formula I (calculated as solvent-free base) and hydroxypropyl cellulose in a weight ratio of 1:0.5 to 1:20.

Another aspect of the invention of particular interest are solid dispersions containing croscarmellose sodium, sodium starch glycolate, crospovidone, low substituted hydroxypropyl cellulose (L-HPC), starch, microcrystalline cellulose or a combination thereof as carrier or disintegrant.

In a preferred embodiment, the solid dispersion comprises polyvinylpyrrolidone and croscarmellose sodium.

In another preferred embodiment, the solid dispersion comprises polyvinylpyrrolidone and sodium starch glycolate.

In another preferred embodiment, the solid dispersion comprises polyvinylpyrrolidone, croscarmellose sodium and microcrystalline cellulose.

In another preferred embodiment, the solid dispersion comprises hydroxypropyl cellulose and croscarmellose sodium.

In yet another preferred embodiment, the solid dispersion comprises hydroxypropyl cellulose and at least one excipient, which is a sugar, sugar alcohol, cyclodextrin.

The solid dispersion of the invention is prepared according to methods known to the art for the manufacture of solid dispersions, such as fusion/melt technology, hot melt extrusion, hot melt coating, prilling, congealing, solvent evaporation (e.g. freeze drying, spray drying, vacuum drying, layering of powders, granules or pellets and fluid bed granulation), coprecipitation, supercritical fluid technology and electrostatic spinning method.

Hot melt extrusion or solvent evaporation techniques are preferred processes for preparation of solid dispersion formulations of this invention.

A solvent suitable for manufacture of solid dispersions by solvent evaporation processes such as spray-drying, layering and fluid-bed granulation can be any compound, wherein the compound of Formula I can be dissolved. Preferred solvents include alcohols (e.g. methanol, ethanol, n-propanol, isopropanol, and butanol), ketones (e.g. acetone, methyl ethyl ketone and methyl isobutyl ketone), esters (e.g. ethyl acetate and propyl acetate) and various other solvents such as acetonitrile, methylene chloride, choroform, hexane, toluene, tetrahydrofurane, cyclic ethers, and 1,1,1-trichloroethane. Lower volatility solvents, such as dimethyl acetamide or dimethylsulfoxide can also be used. Mixtures of solvents, such as 20% ethanol and 80% acetone, can also be used, as can mixtures with water as long as the drug and if necessary the matrix agent are sufficiently soluble to make the process practicable.

In a preferred embodiment the solvent used for manufacture of the solid dispersion comprises methanol, ethanol, n-propanol, isopropanol or acetone.

In a preferred embodiment, a mixture of ethanol and acetone is used as solvent for preparation of the solid dispersion.

An aspect of the invention of particular interest is a composition, wherein the solid dispersion is substantially homogeneous.

An aspect of the invention of particular interest is a pharmaceutical composition, in which the compound of Formula I is substantially amorphous.

This pharmaceutical composition will be utilized to achieve the desired pharmacological effect by oral administration to a patient in need thereof, and will be advantageous to a conventional formulation in terms of drug release, bioavailability, inter-patient variability and/or efficacy in mammals. A patient, for the purpose of this invention, is a mammal, including a human, in need of treatment for the particular condition or disease, including prophylactic treatment.

For oral administration, the solid dispersion described herein can be formulated into solid or liquid preparations such as powder, granules, pellets, tablets, capsules, dragées, chewable tablets, effervescent tablets, dispersible tablets, troches, lozenges, melts, solutions, suspensions, or emulsions, and may be prepared according to methods known to the art for the manufacture of pharmaceutical compositions. For this purpose the solid dispersion may be compounded with conventional excipients, for example binders, fillers, lubricants, disintegrants, solvents, surfactants, emulsifiers, thickeners and stabilizers, glidants and lubricants, coating materials as well as sweeteners, flavoring and coloring agents.

It is believed that one skilled in the art, utilizing the preceding information, can utilize the present invention to its fullest extent. The oral formulation of the compound of Formula I refers to a wide range of dosages such as 1 mg, 10 mg, 100 mg, or even 1 g daily dosing and beyond. This would be accomplished, for example, by modifying the composition and size of the tablet or capsule, and/or by administering multiple tablets or capsules per day to the patient in need thereof. Alternatively, the solid dispersion formulation may also be dosed in forms such as powders, granules, chewable, effervescent or dispersible tablets, or by dispersions of any adequate solid formulation in a suitable liquid prior to use, for example if the optimal dose regimen was no longer consistent with a feasible tablet or capsule size.

### Method of treating hyper-proliferative disorders

The present invention also relates to a method for using a new oral pharmaceutical composition of the compound of Formula I to treat mammalian hyper-proliferative disorders, including cancer. This method comprises administering the pharmaceutical composition in the form of a solid dispersion to a mammal in need thereof, including a human, an amount which is effective to treat the disorder. The term "hyper-proliferative disorders" and/or "cancer" not only refers to solid tumors, such as cancers of the breast, respiratory tract, brain, reproductive organs, digestive tract, urinary tract, eye, liver, skin, head and neck, thyroid, parathyroid and their distant metastases, but also includes lymphomas, sarcomas, and leukemias.

Examples of breast cancer include, but are not limited to invasive ductal carcinoma, invasive lobular carcinoma, ductal carcinoma *in situ,* and lobular carcinoma in situ.

Examples of cancers of the respiratory tract include, but are not limited to small-cell and non-small-cell lung carcinoma, as well as bronchial adenoma and pleuropulmonary blastoma.

Examples of brain cancers include, but are not limited to brain stem and hypophtalmic glioma, cerebellar and cerebral astrocytoma, medulloblastoma, ependymoma, as well as neuroectodermal and pineal tumor.

Tumors of the male reproductive organs include, but are not limited to prostate and testicular cancer. Tumors of the female reproductive organs include, but are not limited to endometrial, cervical, ovarian, vaginal, and vulvar cancer, as well as sarcoma of the uterus.

Tumors of the digestive tract include, but are not limited to anal, colon, colorectal, esophageal, gallbladder, gastric, pancreatic, rectal, small intestine, and salivary gland cancers.

Tumors of the urinary tract include, but are not limited to bladder, penile, kidney, renal pelvis, ureter, and urethral cancers.

Eye cancers include, but are not limited to intraocular melanoma and retinoblastoma.

Examples of liver cancers include, but are not limited to hepatocellular carcinoma (liver cell carcinomas with or without fibrolamellar variant), cholangiocarcinoma (intrahepatic bile duct carcinoma), and mixed hepatocellular cholangiocarcinoma.

Skin cancers include, but are not limited to squamous cell carcinoma, Kaposi's sarcoma, malignant melanoma, Merkel cell skin cancer, and non-melanoma skin cancer.

Head-and-neck cancers include, but are not limited to laryngeal / hypopharyngeal /nasopharyngeal / oropharyngeal cancer, and lip and oral cavity cancer.

Lymphomas include, but are not limited to AIDS-related lymphoma, non-Hodgkin's lymphoma, cutaneous T-cell lymphoma, Hodgkin's disease, and lymphoma of the central nervous system.

Sarcomas include, but are not limited to sarcoma of the soft tissue, fibrosarcoma, osteosarcoma, malignant fibrous histiocytoma, lymphosarcoma, and rhabdomyosarcoma.

Leukemias include, but are not limited to acute myeloid leukemia, acute lymphoblastic leukemia, chronic lymphocytic leukemia, chronic myelogenous leukemia, and hairy cell leukemia.

These disorders have been well characterized in humans, but also exist with a similar etiology in other mammals, such as canines and felines, and can be treated by administering the pharmaceutical compositions of the present invention.

The total amount of the active ingredient (compound of Formula I) to be administered via the oral route using the pharmaceutical composition of the present invention will generally range from about 0.01 mg/kg to about 50 mg/kg body weight per day. Based upon standard laboratory techniques known to evaluate compounds useful for the treatment of hyper-proliferative disorders, by standard toxicity tests and by standard pharmacological assays for the determination of treatment of the conditions identified above in mammals, and by comparison of these results with the results of known medicaments that are used to treat these conditions, the effective dosage of the pharmaceutical compositions of this invention can readily be determined by those skilled in the art. The amount of the administered active ingredient can vary widely according to such considerations as the particular compound and dosage unit employed, the mode and time of administration, the period of treatment, the age, sex, and general condition of the patient treated, the nature and extent of the condition treated, the rate of drug metabolism and excretion, the potential drug combinations and drug-drug interactions, and the like.

The pharmaceutical compositions of this invention can be administered as the sole agent or in combination with one or more other therapies where the combination causes no unacceptable adverse effects. For example, they can be combined with cytotoxic agents, signal transduction inhibitors, or with other anti-cancer agents or therapies, as well as with admixtures and combinations thereof.

In one embodiment, the pharmaceutical compositions of the present invention can be combined with cytotoxic anti-cancer agents. Examples of such agents can be found in the 11th Edition of the Merck Index (1996). These agents include, by no way of limitation, asparaginase, bleomycin, carboplatin, carmustine, chlorambucil, cisplatin, colaspase, cyclophosphamide, cytarabine, dacarbazine, dactinomycin, daunorubicin, doxorubicin (adriamycine), epirubicin, etoposide, 5-fluorouracil, hexamethylmelamine, hydroxyurea, ifosfamide, irinotecan, leucovorin, lomustine, mechlorethamine, 6-mercaptopurine, mesna, methotrexate, mitomycin C, mitoxantrone, prednisolone, prednisone, procarbazine, raloxifen, streptozocin, tamoxifen, thioguanine, topotecan, vinblastine, vincristine, and vindesine.

Other cytotoxic drugs suitable for use with the pharmaceutical compositions of the invention include, but are not limited to, those compounds acknowledged to be used in the treatment of neoplastic diseases in Goodman and Gilman's The Pharmacological Basis of Therapeutics (Ninth Edition, 1996, McGraw-Hill). These agents include, by no way of limitation, aminoglutethimide, L-asparaginase, azathioprine, 5-azacytidine cladribine, busulfan, diethylstilbestrol, 2', 2'-difluorodeoxycytidine, docetaxel, erythrohydroxynonyladenine, ethinyl estradiol, 5-fluorodeoxyuridine, 5-fluorodeoxyuridine monophosphate, fludarabine phosphate, fluoxymesterone, flutamide, hydroxyprogesterone caproate, idarubicin, interferon, medroxyprogesterone acetate, megestrol acetate, melphalan, mitotane, paclitaxel, pentostatin, N-phosphonoacetyl-L-aspartate (PALA), plicamycin, semustine, teniposide, testosterone propionate, thiotepa, trimethylmelamine, uridine, and vinorelbine.

Other cytotoxic anti-cancer agents suitable for use in combination with the compositions of the invention also include newly discovered cytotoxic principles such as oxaliplatin, gemcitabine, capecitabine, epothilone and its natural or synthetic derivatives, temozolomide (Quinn et al., J. Clin. Oncology 2003, 21(4), 646-651), tositumomab (Bexxar), trabedectin (Vidal et al., Proceedings of the American Society for Clinical Oncology 2004, 23, abstract 3181), and the inhibitors of the kinesin spindle protein Eg5 (Wood et al., Curr. Opin. Pharmacol. 2001, 1, 370-377).

In another embodiment, the pharmaceutical compositions of the present invention can be combined with other signal transduction inhibitors. Of particular interest are signal transduction inhibitors which target the EGFR family, such as EGFR, HER-2, and HER-4 (Raymond et al., Drugs 2000, 60 (Suppl.1), 15-23; Harari et al., Oncogene 2000, 19 (53), 6102-6114), and their respective ligands. Examples of such agents include, by no way of limitation, antibody therapies such as Herceptin (trastuzumab), Erbitux (cetuximab), and pertuzumab. Examples of such therapies also include, by no way of limitation, small-molecule kinase inhibitors such as ZD-1839 / Iressa (Baselga et al., Drugs 2000, 60 (Suppl. 1), 33-40), OSI-774 / Tarceva (Pollack et al. J. Pharm. Exp. Ther. 1999, 291(2), 739-748), CI-1033 (Bridges, Curr. Med. Chem. 1999, 6, 825-843), GW-2016 (Lackey et al., 92nd AACR Meeting, New Orleans, March 24-28, 2001, abstract 4582*),* CP-724,714 (Jani et al., Proceedings of the American Society for Clinical Oncology 2004, 23, abstract 3122), AEE-788 (Baselga et al., Proceedings of the American Society for Clinical Oncology 2005, 24, abstract 3028), HKI-272 (Rabindran et al., Cancer Res. 2004, 64, 3958-3965), and EKB-569 (Greenberger et al., 11th NCI-EORTC-AACR Symposium on New Drugs in Cancer Therapy, Amsterdam, November 7-10, 2000, abstract 388*).*

In another embodiment, the pharmaceutical compositions of the present invention can be combined with other signal transduction inhibitors targeting receptor kinases of the split-kinase domain families (VEGFR, FGFR, PDGFR, flt-3, c-kit, c-fms, and the like), and their respective ligands. These agents include, by no way of limitation, antibodies such as Avastin (bevacizumab). These agents also include, by no way of limitation, small-molecule inhibitors such as STI-571 / Gleevec (Zvelebil, Curr. Opin. Oncol., Endocr. Metab. Invest. Drugs 2000, 2(1), 74-82), PTK-787 (Wood et al., Cancer Res. 2000, 60(8), 2178-2189), SU-11248 (Demetri et al., Proceedings of the American Society for Clinical Oncology 2004, 23, abstract 3001), ZD-6474 (Hennequin et al., 92nd AACR Meeting, New Orleans, March 24-28, 2001, abstract 3152), AG-13736 (Herbst et al., Clin. Cancer Res. 2003, 9, 16 (suppl 1), abstract C253), KRN-951 (Taguchi et al., 95th AACR Meeting, Orlando, FL, 2004, abstract 2575), CP-547,632 (Beebe et al., Cancer Res. 2003, 63, 7301-7309), CP-673,451 (Roberts et al., Proceedings of the American Association of Cancer Research 2004, 45, abstract 3989), CHIR-258 (Lee et al., Proceedings of the American Association of Cancer Research 2004, 45, abstract 2130), MLN-518 (Shen et al., Blood 2003, 102, 11, abstract 476), AMG-706 (Herbst et al., Eur. J. Cancer 2004, 2(8), abstract 151). BIBF-1120 (Mross et al., Proceedings of the American Society for Clinical Oncology 2005, 24, abstract 3031), ABT-869 (Albert et al., Proceedings of the American Association of Cancer Research 2005, 46, abstract 676), and AZD-2171 (Hennequin et al., Proceedings of the American Association of Cancer Research 2004, 45, abstract 4539).

In another embodiment, the pharmaceutical compositions of the present invention can be combined with inhibitors of aurora kinases (Mortlock et al., Curr. Topics Med. Chem. 2005, 5, 199). These include, by no way of limitation, VX-680 (Harrington et al., Nature Med. 2004, 10, 262), and PHA-680632 (Fancelli et al., Proceedings of the American Association of Cancer Research 2005, 46, abstract LB-113).

In another embodiment, the pharmaceutical compositions of the present invention can be combined with inhibitors of bcr-abl and/or src. These include, by no way of limitation, AZD-0530 (Gallagher et al., Proceedings of the American Association of Cancer Research 2005, 46, abstract 3972), AMN-107 and BMS-354825 (O'Hare et al., Cancer Res. 2005, 65(11), 4500).

In another embodiment, the pharmaceutical compositions of the present invention can be combined with inhibitors of the Raf/MEK/ERK transduction pathway (Avruch et al., Recent Prog. Horm. Res. 2001, 56, 127-155), or the PKB (akt) pathway (Lawlor et al., J. Cell Sci. 2001, 114, 2903-2910). These include, by no way of limitation, PD-325901 (Sebolt-Leopold et al., Proceedings of the American Association of Cancer Research 2004, 45, abstract 4003), and ARRY-142886 (Wallace et al., Proceedings of the American Association of Cancer Research 2004, 45, abstract 3891).

In another embodiment, the pharmaceutical compositions of the present invention can be combined with inhibitors of histone deacetylase. Examples of such agents include, by no way of limitation, suberoylanilide hydroxamic acid (SAHA), LAQ-824 (Ottmann et al., Proceeding of the American Society for Clinical Oncology 2004, 23, abstract 3024), LBH-589 (Beck et al., Proceedings of the American Society for Clinical Oncology 2004, 23, abstract 3025), MS-275 (Ryan et al., Proceedings of the American Association of Cancer Research 2004, 45, abstract 2452), and FR-901228 (Piekarz et al., Proceedings of the American Society for Clinical Oncology 2004, 23, abstract 3028).

In another embodiment, the pharmaceutical compositions of the present invention can be combined with other anti-cancer agents such as proteasome inhibitors, and m-TOR inhibitors. These include, by no way of limitation, bortezomib (Mackay et al., Proceedings of the American Society for Clinical Oncology 2004, 23, Abstract 3109), and CCI-779 (Wu et al., Proceedings of the American Association of Cancer Research 2004, 45, abstract 3849).

Generally, the use of cytotoxic and/or cytostatic anti-cancer agents in combination with the pharmaceutical compositions of the present invention will serve to:
(1) yield better efficacy in reducing the growth of a tumor or even eliminate the tumor as compared to administration of either agent alone,
(2) provide for the administration of lesser amounts of the administered agents,
(3) provide for a chemotherapeutic treatment protocol that is well tolerated in the patient with fewer deleterious pharmacological complications than observed with single agent chemotherapies and certain other combined therapies,
(4) provide for treating a broader spectrum of different cancer types in mammals, especially humans,
(5) provide for a higher response rate among treated patients,
(6) provide for a longer survival time among treated patients compared to standard chemotherapy treatments,
(7) provide a longer time for tumor progression, and/or
(8) yield efficacy and tolerability results at least as good as those of the agents used alone, compared to known instances where other cancer agent combinations produce antagonistic effects.

It is believed that one skilled in the art, using the preceding information and information available in the art, can utilize the present invention to its fullest extent.

It should be apparent to one of ordinary skill in the art that changes and modifications can be made to this invention without departing from the spirit or scope of the invention as it is set forth herein.

All publications, applications and patents cited above and below are incorporated herein by reference.

A synthetic preparation of the compound used in this invention is described in Example 1. Representative salts of the compound of Example 1 are described in Examples 2, 3, and 4. Novel solid dispersion formulations of the compound of Formula I are described in Example 5 to 20. The examples will serve to further illustrate the invention without limiting it.

### Examples

Abbreviations used in this specification are as follows:

| | |
|---|---|
| HPLC | high pressure liquid chromatography |
| LC-MS | Liquid chromatography-coupled mass spectroscopy |
| LC RT | liquid chromatography retention time |
| MP | melting point |
| NMR | nuclear resonance spectroscopy |
| TLC | thin layer chromatography |
| RT | retention time |
| ES | electrospray |
| HRMS | high resolution mass spectroscopy |

### Preparation of 4-amino-3-fluorophenol

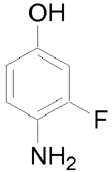

To a dry flask purged with Argon was added 10% Pd/C (80 mg) followed by 3-fluoro-4-nitrophenol (1.2 g, 7.64 mmol) as a solution in ethyl acetate (40 mL). The mixture was stirred under an H₂ atmosphere for 4 h. The mixture was filtered through a pad of Celite and the solvent was evaporated under reduced pressure to afford the desired product as a tan solid (940 mg, 7.39 mmol; 97 % yield); **¹H-NMR** (DMSO-*d₆*) 4.38 (s, 2H), 6.29-6.35 (m, 1H), 6.41 (dd, *J*=2.5, 12.7, 1H), 6.52-6.62 (m, 1H), 8.76 (s, 1H).

### Preparation of 4-(4-amino-3-fluorophenoxy)pyridine-2-carboxylic acid methylamide

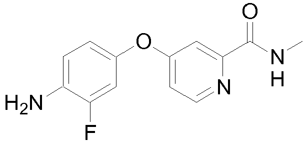

A solution of 4-amino-3-fluorophenol (500 mg, 3.9 mmol) in *N,N*-dimethylacetamide (6 mL) cooled to 0 °C was treated with potassium *tert*-butoxide (441 mg, 3.9 mmol), and the brown solution was allowed to stir at 0 °C for 25 min. To the mixture was added 4-chloro-*N*-methyl-2-pyridinecarboxamide (516 mg, 3.0 mmol) as a solution in dimethylacetamide (4 mL). The reaction was heated at 100 °C for 16 h. The mixture was cooled to room temperature, quenched with H₂O (20 mL), and extracted with ehtylacetate (4 x 40 mL). The combined organics were washed with H₂O (2 x 30 mL), dried (MgSO₄), and evaporated to afford a red-brown oil. ¹H-NMR indicated the presence of residual dimethylacetamide, thus the oil was taken up in diethylether (50 mL) and was further washed with brine (5 x 30 mL). The organic layer was dried (MgSO₄) and concentrated to give 950 mg of the desired product as a red-brown solid, which was used in the next step without purification.

### Example 1: Preparation of 4{4-[3-(4-chloro-3-trifluoromethylphenyl)-ureido]-3-fluorophenoxyl-pyridine-2-carboxylic acid methylamide

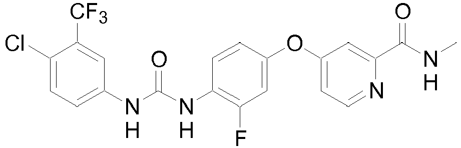

To a solution of 4-(4-amino-3-fluorophenoxy)pyridine-2-carboxylic acid methylamide (177 mg, 0.68 mmol) in toluene (3 mL) was added 4-chloro-3-(trifluoromethyl)phenyl isocyanate (150 mg, 0.68 mmol). The mixture was stirred at room temperature for 72 h. The reaction was concentrated under reduced pressure and the residue was triturated with diethylether. The resulting solid was collected by filtration and dried *in vacuo* for 4 h to afford the title compound (155 mg, 0.32 mmol; 47% yield); **¹H-NMR** (DMSO-*d₆*) 2.78 (d, *J*=4.9, 3H), 7.03-7.08 (m, 1H), 7.16 (dd, *J*=2.6, 5.6, 1H), 7.32 (dd, *J*=2.7, 11.6, 1H), 7.39 (d, *J*=2.5, 1H), 7.60 (s, 2H), 8.07-8.18 (m, 2H), 8.50 (d, *J*=5.7, 1H), 8.72 (s, 1H), 8.74-8.80 (m, 1H), 9.50 (s, 1H); **MS** (HPLC/ES) 483.06 m/z = (M + 1).

A method of preparing 4-chloro-*N*-methyl-2-pyridinecarboxamide is described in Bankston et al. "A Scaleable Synthesis of BAY 43-9006: A Potent Raf Kinase Inhibitor for the Treatment of Cancer" Org. Proc. Res. Dev. 2002, 6(6), 777-781.

### Example 2: Preparation of 4{4-[3-(4-chloro-3-trifluoromethylphenyl)-ureido]-3-fluorophenoxyl-pyridine-2-carboxylic acid methylamide hydrochloride

The compound of Example 1 as a free base (2.0 g) was dissolved in anhydrous tetrahydrofuran (15 mL) and a 4M HCl/dioxane was added (excess). The solution was then concentrated *in vacuo* to afford 2.32 grams of off-white solids. The crude salt was dissolved in hot ethanol (125 mL), activated carbon was added and the mixture heated at reflux for 15 minutes. The hot suspension was filtered through a pad of Celite 521 and allowed to cool to room temperature. The flask was placed in a freezer overnight. The crystalline solids were collected by suction filtration, washed with ethanol, then hexane and air-dried. The mother liquors were concentrated down and crystallization (in freezer) allowed taking place overnight. A second crop of solids was collected and combined with the first crop. The colorless salt was dried in a vacuum oven at 60 °C over two days. Yield of hydrochloride salt obtained 1.72 g (79%).

Melting point: 215 °C

Elemental analysis:

| | Calcd. | Found |
|---|---|---|
| C | 48.57 | 48.68 |
| H | 3.11 | 2.76 |
| N | 10.79 | 10.60 |
| Cl | 13.65 | 13.63 |
| F | 14.63 | 14.88 |

### Example 3: Preparation of 4{4-[3-(4-chloro-3-trifluoromethylphenyl)-ureido]-3-fluorophenoxy}-pyridine-2-carboxylic acid methylamide mesylate

The compound of Example 1 as a free base (2.25 g) was dissolved in ethanol (100 mL) and a stock solution of methanesulfonic acid (excess) was added. The solution was then concentrated *in vacuo* to afford a yellow oil. Ethanol was added and concentration repeated, affording 2.41 g of off-white solids. The crude salt was dissolved in hot ethanol (-125 mL) and then cooled slowly to crystallize. After reaching room temperature, the flask was placed in a freezer overnight. The colorless crystalline material was collected by suction filtration; the filter cake was washed with ethanol, then hexane and air-dried, to afford 2.05 g of material, which was dried in a vacuum oven at 60 °C overnight.

Melting point: 231 °C

Elemental analysis:

| | Calcd. | Found |
|---|---|---|
| C | 45.64 | 45.34 |
| H | 3.31 | 3.08 |
| N | 9.68 | 9.44 |
| Cl | 6.12 | 6.08 |
| F | 13.13 | 13.42 |
| S | 5.54 | 5.59 |

### Example 4: Preparation of 4{4-[3-(4-chloro-3-trifluoromethylphenyl)-ureido]-3-fluorophenoxy}-pyridine-2-carboxylic acid methylamide phenylsulfonate

The compound of Example 1 as a free base (2.25 g) was suspended in ethanol (50 mL) and benzensulfonic acid (0.737 g) in ethanol (50 mL) was added. The mixture was heated with vigorous stirring. All solid material dissolved to give a reddish solution. The solution was allowed to cool to room temperature and the flask scratched. Crystal formation was slow, some seeds were found, added to solution and placed in freezer overnight. Grayish-tan solids had formed in the flask; the material was broken up & collected by suction filtration. The solids were washed with ethanol, then hexane and air-dried. Weighed product: 2.05 g, 69% yield.

Melting point: 213 °C

Elemental Analysis:

| | Calcd. | Found |
|---|---|---|
| C | 50.59 | 50.24 |
| H | 3.30 | 3.50 |
| N | 8.74 | 8.54 |
| F | 11.86 | 11.79 |
| Cl | 5.53 | 5.63 |
| S | 5.00 | 5.16 |

### Example 5: Preparation of a 1+4 solid dispersion of 4{4-[3-(4-chloro-3-trifluoromethylphenyl)-ureido]-3-fluorophenoxy}-pyridine-2-carboxylic acid methyl amide with polyvinylpyrrolidone.

In an uncapped vial, one part of the compound of Example 1 as a free base was mixed with four parts polyvinylpyrrolidone (PVP-25 / Kollidon^{®} 25), and dissolved in a sufficient amount of a 1:1 mixture of acetone and ethanol, until all powders are in solution. The uncapped vial was placed into a vacuum oven set at 40°C, and let dry for at least 24-48 hours.

### Example 6: Preparation of a 1+3 solid dispersion of 4{4-[3-(4-chloro-3-trifluoromethylphenyl)-ureido]-3-fluorophenoxy}-pyridine-2-carboxylic acid methyl amide with polyvinylpyrrolidone.

One part of the compound of Formula I as base and three parts of polyvinylpyrrolidone (PVP 25 / Kollidon^{®} 25) were dissolved in 30 parts of a 80:20 acetone/ethanol mixture (w/w). Using a rotary vacuum evaporator the solvent was removed at 70°C. The dry residue was removed from the evaporation flask and sieved (630 µm).

### Example 7: Preparation of a 1+7 solid dispersion of 4{4-[3-(4-chloro-3-trifluoromethylphenyl)-ureido]-3-fluorophenoxy}-pyridine-2-carboxylic acid methyl 1 amide with polyvinylpyrrolidone.

One part of the compound of Formula I as base and seven parts PVP 25 were dissolved in 30 parts of a 80:20 acetone/ethanol mixture (w/w). Using a rotary vacuum evaporator the solvent was removed at 70°C. The dry residue was removed from the evaporation flask and sieved (630 µm).

### Example 8: Solid dispersion of 4{4-[3-(4-chloro-3-trifluoromethylphenyl)-ureido]-3-fluorophenoxy}-pyridine-2-carboxylic acid methyl amide with hydroxypropyl cellulose (HPC) prepared by melt extrusion.

Two parts of the compound of Formula I as base were mixed with one part of Maltitol and seven parts of HPC-M. The mixture was extruded using a lab twin screw extruder at a temperature of 160-200°C. The extruded material was cut and subsequently milled using an impact lab mill. The resulting powder can be used as it is or it can be further formulated for example to sachet, capsule or tablet formulations.

### Example 9: Solid dispersion of 4{4-[3-(4-chloro-3-trifluoromethylphenyl)-ureido]-3-fluorophenoxy}-pyridine-2-carboxylic acid methyl amide with PVP and croscarmellose sodium.

A solution of 0.4 kg of the of the compound of Formula I as base and 1.2 kg of PVP 25 in a mixture of 6.4 kg acetone and 1.6 kg ethanol was prepared. Using a fluidized bed vacuum granulator this solution was sprayed onto a powder bed of 1.6 kg croscarmellose sodium at a temperature of 60-70°C. After drying the product was sieved (1 mm). The granulate can be used as it is or it can be further formulated for example to sachet, capsule or tablet formulations.

### Example 10: Solid dispersion of 4{4-[3-(4-chloro-3-trifluoromethylphenyl)-ureido]-3-fluorophenoxyl-pyridine-2-carboxylic acid methyl amide with PVP and sodium starch glycolate.

This material was prepared in a similar way as described in Example 9, except that the solution is sprayed onto a powder bed of 1.6 kg sodium starch gycolate Type A (Explotab^{®})

### Example 11: Solid dispersion of 4{4-[3-(4-chloro-3-trifluoromethylphenyl)-ureido]-3-fluorophenoxyl-pyridine-2-carboxylic acid methyl amide with PVP and croscarmellose sodium.

A solution of 0.4 kg of the of the compound of Formula I as base and 1.6 kg of PVP 25 in a mixture of 6.4 kg acetone and 1.6 kg ethanol was prepared. Using a fluidized bed vacuum granulator this solution was sprayed onto a powder bed of 2 kg croscarmellose sodium at a temperature of 60-70°C. After drying the product was sieved (1 mm). The granulate can be used as it is or it can be further formulated for example to sachet, capsule or tablet formulations.

### Example 12: Solid dispersion of 4{4-[3-(4-chloro-3-trifluoromethylphenyl)-ureido]-3-fluorophenoxyl-pyridine-2-carboxylic acid methyl amide with PVP, croscarmellose sodium and microcrystalline cellulose.

This material was prepared in a similar way as described in Example 11, except that the solution was sprayed onto a powder bed consisting of 1 kg croscarmellose sodium and 1 kg microcrystalline cellulose.

### Example 13: Solid dispersion of 4{4-[3-(4-chloro-3-trifluoromethylphenyl)-ureido]-3-fluorophenoxy}-pyridine-2-carboxylic acid methyl amide with HPC-SL and croscarmellose sodium.

A solution of 0.4 kg of the of the compound of Formula I as base and 1.6 kg of HPC-SL in 20 kg acetone was prepared. Using a fluidized bed vacuum granulator this solution was sprayed onto a powder bed of 2 kg croscarmellose sodium at a temperature of 40-60°C. After drying the product was sieved (1 mm). The granulate can be used as it is or it can be further formulated for example to sachet, capsule or tablet formulations.

### Example 14: Solid dispersion of 4{4-[3-(4-chloro-3-trifluoromethylphenyl)-ureido]-3-fluorophenoxyl-pyridine-2-carboxylic acid methyl amide with HPC-L and croscarmellose sodium.

A solution of 0.4 kg of the of the compound of Formula I as base and 1.6 kg of HPC-L in 28 kg acetone was prepared. Using a fluidized bed vacuum granulator this solution was sprayed onto a powder bed of 2 kg croscarmellose sodium at a temperature of 40-60°C. After drying the product was sieved (1 mm). The granulate can be used as it is or it can be further formulated for example to sachet, capsule or tablet formulations.

### Example 15: Tablets containing a solid dispersion of 4{4-[3-(4-chloro-3-trifluoromethylphenyl)-ureido]-3-fluorophenoxy}-pyridine-2-carboxylic acid methyl amide

The granulate of Example 11 was roller compacted and screened 3 and 1 mm. Subsequently the compacted granulate was blended with 0.54 kg croscarmellose sodium, 24 g colloidal anhydrous silica and 36 g magnesium stearate. This ready-to-press blend was compressed on a rotary tablet press to tablets containing 20, 50 an 100 mg of the compound of Formula I. The tablets may be film-coated for light protection.

### Example 16: Tablets containing a solid dispersion of 4{4-[3-(4-chloro-3-trifluoromethylphenyl)-ureido]-3-fluorophenoxy}-pyridine-2-carboxylic acid methyl amide

The granulate of Example 12 was roller compacted and screened 3 and 1 mm. Subsequently the compacted granulate was blended with 0.54 kg croscarmellose sodium, 24 g colloidal anhydrous silica and 36 g magnesium stearate. This ready-to-press blend was compressed on a rotary tablet press to tablets containing 20, 50 an 100 mg of the compound of Formula I. The tablets may be film-coated for light protection.

### Example 17: Tablets containing a solid dispersion of 4{4-[3-(4-chloro-3-trifluoromethylphenyl)-ureido]-3-fluorophenoxy}-pyridine-2-carboxylic acid methyl amide

A solution of 0.4 kg of the of the compound of Formula I as base and 1.2 kg of PVP 25 in a mixture of 6.4 kg acetone and 1.6 kg ethanol was prepared. Using a fluidized bed vacuum granulator this solution was sprayed onto a powder bed consisting of 0.8 kg croscarmellose sodium and 0.8 kg microcrystalline cellulose at a temperature of 60-70°C. After drying the product is sieved (1 mm). The granulate is roller compacted and screened 3 and 1 mm. Subsequently the compacted granulate was blended with 1.34 kg croscarmellose sodium, 24 g colloidal anhydrous silica and 36 g magnesium stearate. This ready-to-press blend is compressed on a rotary tablet press to tablets containing 20, 50 an 100 mg of the compound of Formula I. The tablets may be film-coated for light protection.

### Example 18: Comparison of the drug dissolution of new pharmaceutical compositions and a conventional formulation of the compound of Formula I.

Drug dissolution of the new solid dispersion formulations of Example 6 and 7 was studied using the paddle apparatus "Apparatus 2" of the USP 28-NF 23 (The United States Pharmacopoeia USP 28 2005) at a temperature of 37 ± 0.5°C and a paddle speed of 75 rpm. 900 mL of acetate buffer pH 4.5 USP with the addition of 0.1% (m/V) sodium lauryl sulfate as surfactant was used as dissolution medium. An amount of solid dispersion equivalent to 50 mg of the compound of Formula I as base was inserted in each vessel. During the dissolution test samples were drawn through a filter and the dissolved amount of drug substances was determined by UV spectrometry. The determined amount of active was calculated as % (m/m) of total dose (50 mg).

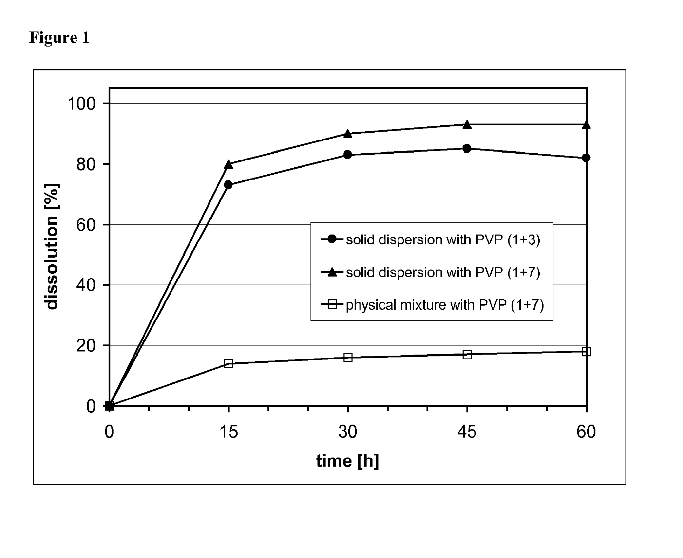

In Figure 1 the drug dissolution of the solid dispersion of Example 6 and 7 was compared to a 1+7 physical mixture of the compound of Formula I as base with PVP 25 prepared by blending of the active and the excipient in a Turbula mixer (conventional formulation).

The results demonstrate that drug dissolution of the physical mixture is slow and incomplete. Due to the low solubility of the active only about 20% of the 50 mg dose are dissolved. The solid dispersions, however, show fast and almost complete dissolution of the active. Approximately 90% of the dose, equivalent to ca. 45 mg of the compound of this invention are dissolved within 1 hour, despite the poor solubility of the active in this medium (2.3 mg/900 mL at 37°C). The solid new pharmaceutical compositions of Example 6 and 7, which contain the active in substantially amorphous form, show clear supersaturation compared to the physical mixture of the active and PVP, and no significant recrystallisation is observed for at least 1 hour. Due to the fast dissolution, strong capability to form supersaturated solutions and good stability of the supersaturated solution the new solid dispersion formulations of this invention are rated suitable for achieving improved absorption, bioavailability and efficacy in the treatment of hyper-proliferative disorders, including cancer.

### Example 19: Comparison of the oral bioavailability of several conventional formulations of the compound of Example 1, and the new pharmaceutical composition of Example 5.

A single dose of the compounds of Examples 1, 2, 3, 4, and 5 was administered to each of three fasted male Wistar rats (220-250 g; 6-8 weeks old) orally. Compounds of Examples 1-4 were administered by oral gavage in the formulation described in Table 1. The solid dispersion of Example 5 (1.25 mg drug substance in 5 mg PVP-25) was administered in size 9 capsules. One capsule was administered per rat.

Approximately 0.4 mL of whole blood was collected via an indwelling jugular catheter at: 0.25, 0.5, 1, 2, 4, 7, 24, and 48 h post-dose. The blood samples were centrifuged (approx. 5 min.) in order to obtain plasma which was then transferred to the appropriately labeled vials and stored frozen (-20°C) until analysis for parent drug.

Plasma samples were analyzed via LC/MS/MS for parent drug concentrations and pharmacokinetic parameters were calculated using Watson LIMS.

**Table 1: Comparative oral bioavailabilities in the rat for different formulations of the compound of Formula I**

| Example Number | Dose(m g/kg) | Vehicle | AUC (0-inf)(mg.h/L) | %F |
|---|---|---|---|---|
| **1** | 5 | 0.5% tylose in water | 17 | 10 |
| **1** | 5 | 0.5% NaCMC and 0.5% Tween 80 in water | 21.8 | 13 |
| **2** | 5 | 0.5% NaCMC and 0.5% Tween 80 in water | 12.1 | 7 |
| **3** | 5 | 0.5% NaCMC and 0.5% Tween 80 in water | 16.8 | 10 |
| **4** | 5 | 0.5% NaCMC and 0.5% Tween 80 in water | 15.8 | 10 |
| **5** | 5 | Encapsulated | 42.2 | 25 |

The dose of 5 mg/kg of the pharmaceutical composition of Example 5 means 5 mg of drug substance (compound of Example 1) per kg of body weight. The relative bioavailabilities in the rat are calculated from the data of an intravenous bolus dose of 2 mg/kg in the same strain of rats, using a solution of the active in a Cremophor RH40: ethanol: water (12.5:12.5:75) vehicle. The plasma exposure in the exposure arm of the suspension in 0.5% tylose solution (9.9% F) reflects a crystalline, micronized drug substance with the following characteristics: 208 °C (onset), 211 °C (peak), □H = 91.4 J/g.

The pharmaceutical composition based on a 1+4 solid dispersion of the compound of this invention with polyvinylpyrrolidone (PVP-25, Example 5) provides an improved absorption and oral exposure in the rat, compared to two conventional formulations of the free base, and three conventional formulations of pharmaceutically acceptable salts of the compound of Formula I.

### Example 20: Comparison of the exposure of a new pharmaceutical composition and a conventional formulation of the compound of Formula I

Single doses of the compound of this invention in form of a conventional and a novel formulation were administered orally by gavage to fasted male rats (approx. 250 g; 3 rats per time point).

The conventional formulation was a suspension of the micronized compound of formula I as base in an aqueous 0.5% tylose solution.

The novel formulation was the solid dispersion granulate of Example 11, which was suspended in water prior to administration.

For the conventional formulation doses of 2, 10 and 50 mg drug substance/kg body weight were applied in a volume of 5 mL/kg body weight. For the new composition of Example 11, containing 10% of the compound of this invention, an amount of 20, 100 and 500 mg granulate/kg body weight (equivalent to doses of 2, 10 and 50 mg/kg respectively) was administered in 5 mL water/kg body weight.

Approximately 0.5 mL of whole blood was collected via an indwelling jugular catheter at 0.5, 1, 2, 4, 7, 9, and 24 h post-dose. The blood samples were centrifuged in order to obtain plasma which was then transferred to the appropriately labeled vials and stored frozen (< -15°C) until analysis for parent drug.

Plasma samples were analyzed via LC/MSMS for parent drug concentrations and pharmacokinetic parameters were calculated. The results are presented in Table 2.

**Table 2: Comparison of the exposure in the rat for different formulations of the compound of Formula I**

| | Suspension of micronized compound | | | Solid dispersion suspended in water | | |
|---|---|---|---|---|---|---|
| Dose [mg/kg] | 2 | 10 | 50 | 2 | 10 | 50 |
| AUC [mg*h/L] | 4.1 | 13.9 | 30.1 | 9.8 | 48.6 | 152 |
| Cₘₐₓ [mg/L] | 0.34 | 1.11 | 2.24 | 1.00 | 5.28 | 12.7 |

The AUC and cₘₐₓ data reveal significant differences for the exposure of the active after oral application of the conventional and the novel formulation: the exposure obtained after administration of the new composition of this invention is significantly higher compared to the exposure of the conventional suspension formulation. At doses of 2 and 10 mg/kg AUC is increased by a factor of 2.5 to 3.5 and cₘₐₓ is increased by factor 2.9 respectively 4.8 for the novel composition of this invention. At the highest dose of 50 mg/kg the increase is even more pronounced: both AUC and cₘₐₓ of the novel formulation are 5 times higher than for the conventional suspension.

This proves that even when administering high doses in animals studies, absorption and oral exposure are improved significantly by application of novel compositions of this invention containing the compound of Formula I in the form of a solid dispersion compared to conventional formulations of this drug.

Based on these findings it can be assumed that this new type of pharmaceutical composition, comprising a solid dispersion of the compound of Formula I, will result in improved absorption and exposure, reduced inter-patient variability, and overall superior efficacy for the treatment of hyper-proliferative disorders, including cancer.

Without further elaboration, it is believed that one skilled in the art can, using the preceding description, utilize the present invention to its fullest extent. The preceding preferred specific embodiments are, therefore, to be construed as merely illustrative, and not limitative of the remainder of the disclosure in any way whatsoever.

In the foregoing and in the examples, all temperatures are set forth uncorrected in degrees Celsius and, all parts and percentages are by weight, unless otherwise indicated.

The entire disclosures of all applications, patents and publications, cited herein and of corresponding U.S. Provisional Application Serial No. 60/604,752, filed August 27, 2004, are incorporated by reference herein.

The preceding examples can be repeated with similar success by substituting the generically or specifically described reactants and/or operating conditions of this invention for those used in the preceding examples.

From the foregoing description, one skilled in the art can easily ascertain the essential characteristics of this invention and, without departing from the spirit and scope thereof, can make various changes and modifications of the invention to adapt it to various usages and conditions.

## Claims

1. A composition comprising a solid dispersion comprising at least 4{4-[3-(4-chloro-3-trifluoromethylphenyl)-ureido]-3-fluorophenoxy}-pyridine-2-carboxylic acid methyl amide, its solvates, hydrates, pharmaceutically acceptable salts, or a combination thereof and a pharmaceutically acceptable matrix.

2. A composition according to claim 1, wherein the matrix comprises polymeric excipients or non-polymeric excipients capable of dissolving or dispersing 4{4-[3-(4-chloro-3-trifluoromethylphenyl)-ureido]-3-fluorophenoxy}-pyridine-2-carboxylic acid methyl amide, its solvates, hydrates, pharmaceutically acceptable salts, or a combination thereof.

3. A composition according to claim 1, wherein the matrix comprises a combination of polymeric excipients and non-polymeric excipients capable of dissolving or dispersing 4 {4-[3-(4-chloro-3-trifluoromethylphenyl)-ureido]-3-fluorophenoxy} - pyridine-2-carboxylic acid methyl amide, its solvates, hydrates, pharmaceutically acceptable salts, or a combination thereof.

4. A composition according to claim 1, wherein the matrix comprises a water soluble polymer.

5. A composition according to claim 4, wherein the matrix comprises at least one polymer from the group consisting of polyvinylpyrrolidone, copovidone, hydroxypropyl cellulose, hydroxypropyl methyl cellulose, polyethylene glycole or polyethylene oxide.

6. A composition according to claim 5, wherein polyvinylpyrrolidone is used as matrix agent.

7. A composition according to claim 6, wherein the weight ratio of 4{4-[3-(4-chloro-3-trifluoromethylphenyl)-ureido]-3-fluorophenoxy}-pyridine-2-carboxylic acid methyl amide, its solvates, hydrates, pharmaceutically acceptable salts, or a combination thereof calculated as solvent-free base to polyvinylpyrrolidone is between 1:0.5 and 1:20.

8. A composition according to claim 5, wherein hydroxypropyl cellulose is used as matrix agent.

9. A composition according to claim 8, wherein the weight ratio of 4{4-[3-(4-chloro-3-trifluoromethylphenyl)-ureido]-3-fluorophenoxy}-pyridine-2-carboxylic acid methyl amide, its solvates, hydrates, pharmaceutically acceptable salts, or a combination thereof calculated as solvent-free base to hydroxypropyl cellulose is between 1:0.5 and 1:20.

10. A composition according to any of the proceeding claims, wherein the solid dispersion comprises croscarmellose sodium, sodium starch glycolate, crospovidone, low substituted hydroxypropyl cellulose, starch, microcrystalline cellulose or a combination thereof.

11. A composition according to claim 7, wherein the solid dispersion comprises polyvinylpyrrolidone and croscarmellose sodium.

12. A composition according to claim 7, wherein the solid dispersion comprises polyvinylpyrrolidone and sodium starch glycolate.

13. A composition according to claim 7, wherein the solid dispersion comprises polyvinylpyrrolidone, croscarmellose sodium and microcrystalline cellulose.

14. A composition according to claim 9, wherein the solid dispersion comprises hydroxypropyl cellulose and croscarmellose sodium.

15. A composition according to claim 9, wherein the solid dispersion comprises hydroxypropyl cellulose and at least one excipient which is a sugar, sugar alcohol, cyclodextrin.

16. A composition according to any of the proceeding claims, wherein the solid dispersion is substantially homogeneous.

17. A composition according to any of the proceeding claims, which contains 4{4-[3-(4-chloro-3-trifluoromethylphenyl)-ureido]-3-fluorophenoxy}-pyridine-2-carboxylic acid methyl amide, its solvates, hydrates, pharmaceutically acceptable salts, or a combination thereof in substantially amorphous form.

18. A composition as claimed in any one of the proceeding claims, which is a pharmaceutical composition for oral application.

19. A composition as claimed in any one of the proceeding claims, which is a pharmaceutical composition in form of a tablet.

20. A composition as claimed in any one of the proceeding claims, which is a pharmaceutical composition in form of a capsule.

21. A composition as claimed in any one of the proceeding claims, which is a pharmaceutical composition in form of a powder, granulate or sachet.

22. A process for the preparation of a solid dispersion of 4{4-[3-(4-chloro-3-trifluoromethylphenyl)-ureido]-3-fluorophenoxy}-pyridine-2-carboxylic acid methyl amide, its solvates, hydrates, pharmaceutically acceptable salts, or a combination thereof which comprises simultaneously exposing 4{4-[3-(4-chloro-3-trifluoromethylphenyl)-ureido]-3-fluorophenoxy}-pyridine-2-carboxylic acid methyl amide, its solvates, hydrates, pharmaceutically acceptable salts, or a combination thereof and at least one matrix agent to hot melt extrusion, hot melt coating, prilling, congealing, solvent evaporation techniques or a combination thereof.

23. A process according to claim 22, wherein the solid dispersion is prepared by exposing 4{4-[3-(4-chloro-3-trifluoromethylphenyl)-ureido]-3-fluorophenoxy}-pyridine-2-carboxylic acid methyl amide, its solvates, hydrates, pharmaceutically acceptable salts, or a combination thereof and at least one matrix agent to hot melt extrusion.

24. A process according to claim 22, wherein the solid dispersion is prepared by exposing 4{4-[3-(4-chloro-3-trifluoromethylphenyl)-ureido]-3-fluorophenoxy}-pyridine-2-carboxylic acid methyl amide, its solvates, hydrates, pharmaceutically acceptable salts, or a combination thereof and at least one matrix agent to solvent evaporation techniques.

25. A process according to claim 24, wherein the solvent comprises one or more solvents selected from the group consisting of methanol, ethanol, n-propanol, isopropanol and acetone.

26. A process according to claim 25, wherein a mixture of ethanol and acetone is used as solvent.

27. A process as claimed in claim 22 to 26, wherein the solid dispersion is further treated with at least one additional processing step, which is milling, sieving, roller compaction, grinding, screening, mixing or a combination thereof.

28. A process as claimed in claim 22 to 27 comprising the additional step of compounding the solid dispersion with one or more pharmaceutical acceptable excipients to form a mixture and shaping this mixture into tablets, filled capsules or sachets.

29. A pharmaceutical composition, whenever produced by a process of any of the claims 22 to 28.

30. A pharmaceutical composition according to any of the claims 1 to 21 and 29 for treatment of hyper-proliferative disorders in a mammal, including a human, either as sole agent or in combination with other therapies.

31. A pharmaceutical composition according to any of the claims 1 to 21 and 29 for treatment of cancer ina mammal, including a human, either as sole agent or in combination with other therapies.

32. A pharmaceutical composition according to any of the claims 1 to 22 and 30 for treatment of cancerin a mammal, including a human, either as sole agent or in combination with radio therapy.

33. A pharmaceutical composition according to any of the claims 1 to 21 and 29 for treatment of cancer in a human patient, in combination with a cytotoxic therapy.

34. A pharmaceutical composition according to any of the claims 1 to 21 and 29 for treatment of cancer in a human patient, in combination with another anti-cancer therapy targeting either VEGFR, PDGFR, src, abl flt-3, EGFR, HER-2, aurora, raf, MEK, ERK, PI-3 kinase, AKT, mTOR, or HDAC.
